# EUROPÄISCHE PATENTSCHRIFT

(11) **EP 1 153 572 B1**
(45) Veröffentlichungstag und Bekanntmachung des Hinweises auf die Patenterteilung: **07.08.2002**
(21) Anmeldenummer: 00109193.3
(22) Anmeldetag: 09.05.2000
(51) Int. Cl.: A61B 5/11, A61B 19/00

(54) **Verfahren zur Registrierung eines Patientendatensatzes aus einem bildgebenden Verfahren bei navigationsunterstützen chirurgischen Eingriffen mittels Röntgenbildzuordnung**
Method of registering of patient data of an imaging method for navigation supported surgical operations by means of X-ray image correspondance
Méthode d'enregistrement des donées d'image d'un patient résultants d'une méthode de navigation, pour des opérations chirurgicales avec des rayons X

(43) Veröffentlichungstag der Anmeldung: 14.11.2001
(73) Patentinhaber: BrainLAB AG, 85551 Kirchheim/Heimstetten (DE)
(72) Erfinder: Vilsmeier, Stefan, 6330 Kufstein (AT)
(74) Vertreter: Schwabe - Sandmair - Marx

(56) Entgegenhaltungen:
- EP-A- 0 488 987
- WO-A-98/38908
- US-A- 5 368 030
- US-A- 5 383 454
- US-A- 5 636 255

## Beschreibung

Die vorliegende Erfindung betrifft ein Verfahren zur Registrierung eines Patientendatensatzes aus einem bildgebenden Verfahren bei kameranavigationsunterstützten chirurgischen Eingriffen.

Chirurgische Eingriffe werden in jüngerer Zeit immer öfter unter Zuhilfenahme von sogenannten Navigationssystemen durchgeführt, die es dem behandelnden Arzt gestatten, die Operation bildunterstützt durchzuführen. Solche Navigationssysteme sind beispielsweise in der DE 196 39 615 A1 beschrieben, wo mit einem passiven Reflektorenreferenzierungssystem gearbeitet wird, während aus der US-A-5,383,454 ein Instrumentenpositionierungssystem bekannt ist, das unter Verwendung aktiv abstrahlender, positionsgebender Marker arbeitet.

Solche Navigationssysteme basieren auf einer Scan-Erfassung, welche vor der eigentlichen Behandlung, meist auch an einem anderen Ort als dem eigentlichen Operationssaal und oft in zeitlichem Abstand mit bereits angebrachten Patientenmarkern gemacht wurden. Selbst wenn schon bei einem solchen Patientenscan, beispielsweise einer Computer- oder Kernspinresonanztomographie, Markierungen verwendet werden, die später bei der Behandlung auch vom Navigationssystem erfasst werden können, lassen sich Ungenauigkeiten bei der Vor-Ort-Registrierung im Operationssaal, die aus Verschiebungen der Markierungen oder durch ungenaue Registrierungsmethoden entstehen, nicht vermeiden.

Man hat deshalb versucht, die Registrierung der aktuellen Lage im Operationssaal nochmals durch vor Ort erstellte Röntgenaufnahmen in der Genauigkeit zu verbessern.

So wurde vorgeschlagen, bei Operationen mit Hilfe einer Dauer-Röntgenerfassung zu arbeiten, wobei ein Röntgengerät mit einer Röntgenstrahlungsquelle und einem Bildverstärker kontinuierlich ein Röntgenbild liefert, das, auf einem Bildschirm ausgegeben, dem Arzt bei der Operation visuelle Hilfe gibt. Hierbei entsteht nachteiligerweise durch die andauernde Röntgenbestrahlung eine hohe Strahlungsbelastung und außerdem kann nur eine relativ ungenaue, meist lediglich zweidimensionale Bildunterstützung bereitgestellt werden.

Aus der US-A-4,791,934 ist ein computertomographieunterstütztes, stereotaktisches Chirurgiesystem bekannt, bei dem mittels eines C-Bogen-Röntgengerätes vor der Eingriffsprozedur mehrere zweidimensionale Röntgenbilder erzeugt werden, die dann mit rekonstruierten Bildern aus dem Scan-Datensatz so lange überlagert werden, bis die aktuelle Position mit einer relativ hohen Genauigkeit feststeht, dass heißt der Tomographie-Datensatz im Navigationssystem aktuell registriert ist.

Jedoch führt auch diese Methode noch zu Ungenauigkeiten, da der Patient während eines chirurgischen Eingriffs durchaus noch Bewegungen unterworfen ist. Dabei ist zu bedenken, dass manche chirurgische Eingriffe schon bei der Freilegung der zu behandelnden Bereiche durchaus massive Manipulationen erfordern, welche die Lage bestimmter Patientenstrukturen und insbesondere deren Relativlage trotz aller Fixierungen noch im Zentimeterbereich verändern können.

Mit der Röntgenerfassung gemäß der US-A-4,791,934, die vor dem Eingriff durchgeführt wird, können solche Verschiebungen nicht berücksichtigt werden.

US-A-5 636 255 offenbart ferner ein Verfahren zur CT-Bild Registrierung unter Verwendung von Markeranordnungen.

Es ist die Aufgabe der vorliegenden Erfindung ein Verfahren zur Registrierung eines Patientendatensatzes bereitzustellen, welches die oben genannten Nachteile des Standes der Technik überwindet. Insbesondere soll eine Möglichkeit geschaffen werden, über den gesamten Operationszeitraum eine positionskorrekte Navigation durchzuführen.

Diese Aufgabe wird gelöst durch ein Verfahren zur Registrierung eines Patientendatensatzes aus einem bildgebenden Verfahren bei kameranavigationsunterstützten chirurgischen Eingriffen gemäß Anspruch 1.

Der Hauptvorteil der vorliegenden Erfindung liegt in der Möglichkeit, eine äußerst genaue Navigation durchführen zu können, und zwar zu jedem Zeitpunkt der Behandlung. Alle Bewegungen und Relativbewegungen im zu behandelnden Bereich können über die Verfolgung der Bewegung einer oder mehrerer fester Körperstrukturen erfasst werden, wobei aus der erfassbaren Verschiebung dieser Strukturen auf die Lageänderung umgebender Bereiche, beispielsweise Weichteilbereiche zurückgerechnet werden kann. Damit stehen die Daten des Patientendatensatzes auch dann zur Verfügung, wenn die Lage des zu behandelnden Bereiches und insbesondere die Relativlage von einzelnen Strukturen im zu behandelnden Bereich nicht mehr dieselbe ist wie bei der vorab durchgeführten Erfassung durch das bildgebende Verfahren (Scan).

Durch die Möglichkeit dieser zu jedem Zeitpunkt aktualisierbaren Navigation können Eingriffe gezielter und damit weniger invasiv vorgenommen werden. Auch die Dauer solcher Eingriffe kann sich damit verringern und bleibende Sterilität über die Eingriffszeit wird sichergestellt.

Als Verfahren zur Erstellung des Patientendatensatzes eignet sich im Rahmen der vorliegenden Erfindung jedes bildgebende Verfahren, beispielsweise die Computertomographie (CT), die Kernspintomographie (Magnetresonanztomographie, MRI), PET-, SPECT-Verfahren und die Ultraschallerfassung. Durch die erfindungsgemäße und jederzeit aktualisierbare Registrierung des Patientendatensatzes können grundsätzlich aufwändige Registrierungsverfahren entfallen, wie beispielsweise die einzelne Registrierung von Markierungen auf der Patientenhaut oder auf der Knochenoberfläche durch das Anfahren mit einem im Navigationssystem verfolgbaren Zeigegerät. Damit wird die Erstellung des Patientendatensatzes zeitlich weitgehend von der Behandlung entkoppelt; es kann ohne weiteres mit Patientenscans gearbeitet werden, die Wochen vor dem eigentlichen Eingriff erstellt wurden. Außerdem kann mit jedweden Scan-Datensätzen gearbeitet werden, da die Registrierung nicht mehr von Markierungen abhängig ist, die schon beim Erstellen des Patientendatensatzes durch das bildgebende Verfahren am Patienten angebracht waren.

Bei einer bevorzugten Ausführungsform des erfindungsgemäßen Verfahrens werden an zueinander beweglichen Körperstrukturen jeweils Markeranordnungen angebracht, so dass die Relativbewegungen dieser Strukturen zueinander erfasst werden. Außerdem kann der Bildinhalt der Röntgenaufnahme(n) computergestützt segmentiert und dabei einzelne, insbesondere zueinander bewegliche Strukturen als getrennte Objekte erfasst und eingeordnet bzw. entsprechenden Objekten aus dem Patientendatensatz zugeordnet werden.

Die angesprochene Segmentierung spielt eine wichtige Rolle im Rahmen der Behandlung von Körperbereichen, wo Knochenstrukturen, die mit dem Röntgenbild sehr gut erfassbar sind, einzeln zueinander beweglich sind. Dies ist beispielsweise bei Knochenbrüchen oder einzelnen Wirbeln der Fall, jedoch auch bei ansonsten gelenkig miteinander verbundenen Knochen, wie beispielsweise im Oberschenkel- und Unterschenkelknochen. Erfindungsgemäß werden solche zueinander bewegliche Strukturen als einzelne Segmente diskret erfasst, und bei der Registrierung des Patientendatensatzes wird dann eine Anpassung vorgenommen, so dass die Informationen aus dem Datensatz für die tatsächlich vorhandene, aktuelle Relativlage der Strukturen wieder zur Verfügung stehen. Mit anderen Worten liefert die Positionsinformation über segmentierte zueinander bewegliche Teile eine zusätzliche Information zum Patientendatensatz, die rechnerisch, computergestützt verwertet werden kann, um sicher zu stellen, dass ein solcher Patientendatensatz, der beispielsweise in einer etwas anderen Relativlage der Strukturen erstellt wurde, während der Operation eine aktuell richtige Aussage zur Lage der einzelnen Körperbereiche machen kann. Dabei können nicht nur die unmittelbar erfassbaren Relativbewegungen von festen Strukturen, an denen die Markeranordnungen direkt befestigt sind berücksichtigt werden, sondern es ist möglich auch die Position von umgebendem Gewebe zu berechnen. Dies gilt nicht nur für Änderungen in der Relativlage zwischen Scan-Erfassung und tatsächlichem Eingriffszeitpunkt, sondern auch für Bewegungen, die während des Eingriffs vorkommen oder durch diesen verursacht werden.

Die verwendeten Markeranordnungen können im Rahmen der Erfindung sowohl aktiv abstrahlende Marker als auch Reflexionsmarker umfassen und sie werden beispielsweise über sterile Adapter an Knochenstrukturen, insbesondere auch an Wirbelfortsätzen angebracht.

Die Segmentierung, wie sie oben angesprochen wurde, sowie die Ein- bzw. Zuordnung der getrennten und diskreten Objekte erfolgt vorzugsweise automatisch im Rahmen der computergestützten Navigation bzw. Navigationsaktualisierung mittels der Röntgenaufnahme. Hiezu stehen Algorithmen zur Verfügung.

Eine Möglichkeit, die aktuelle Registrierung des Patientendatensatzes innerhalb des erfindungsgemäßen Verfahrens vorzunehmen, besteht dadurch, die Röntgenaufnahmen mit digital rekonstruierten Röntgenbildern (DRR's) aus dem Patientendatensatz so lange zu vergleichen und abzugleichen, bis eine Situation gefunden ist, in der der angepasste Patientendatensatz mit der tatsächlichen, durch die Röntgenerfassung ermittelten Situation übereinstimmt. Verfahren zur Ermittlung der Raumposition von DRR's innerhalb eines Navigationssystems sind bekannt und können im Rahmen der Erfindung Verwendung finden. Ferner ist es erfindungsgemäß möglich, die aktuelle Registrierung des Patientendatensatzes durch einen Vergleich und Abgleich von Konturen aus der/den Röntgenaufnahme(n) mit Oberflächeninformationen aus dem Patientendatensatz durchzuführen.

Schließlich besteht eine weitere erfindungsgemäße Möglichkeit zur Registrierung des Patientendatensatzes darin, ein im Navigationssystem registrier- und verfolgbares C-Bogen-Röntgengerät zu verwenden, das um eine horizontale Achse drehbar ist und einem isozentrischen Strahlengang aufweist. Bei, der Erstellung von mehreren Bildern aus verschiedenen Winkeln durch ein solches Röntgengerät mit isozentrischem Strahlengang, sind die Bilder am Isozentrum ortsfest bestimmt, und durch eine Positionsfeststellung für das Röntgengerät selbst, beispielsweise durch Navigationsmarker, kann dann die Lage der erstellten Röntgenbilder ermittelt und bei der Registrierung des Patientendatensatzes verwendet werden.

In allen genannten Fällen kann die Zuordnung von Strukturen aus dem Patientendatensatz und der/den Röntgenaufnahme(n) mittels einer dreidimensionalen Bildfusionstechnik über Grauwertabgleich erfolgen.

Damit stellt das erfindungsgemäße Verfahren erstmals die Möglichkeit einer aktuell exakten Navigation zur Verfügung, die unbeeinflusst von Bewegungen bzw. Relativbewegungen (auch während der Operation verursachte) im zu behandelnden Bereich durchgeführt werden kann.

## Patentansprüche

1. Verfahren zur Registrierung eines Patientendatensatzes aus einem bildgebenden Verfahren bei kameranavigationsunterstützten chirurgischen Eingriffen mit den folgenden Schritten:
- ein Patientendatensatz wird mittels eines Tomographie-Scans bzw. eines bildgebenden Verfahrens für den Patientenkörper oder einen Teil davon erstellt,
- nach dem Verbringen des Patienten in einen Operationssaal und bei der Behandlung wird an mindestens einer festen Körperstruktur mindestens eine im Navigationssystem identifizierbare und verfolgbare Markeranordnung angebracht,
- mittels eines Röntgengerätes wird eine oder mehrere Röntgenaufnahmen des zu behandelnden Bereiches erstellt, deren räumliche Position im Navigationssystem ermittelt wird,
- mittels einer computergestützten Zuordnung wird der Patientendatensatz mit den aus der Röntgenaufnahme erhaltenen Positionsdaten aktuell registriert, und
- mittels der Verfolgung der Markeranordnung(en) im Navigationssystem werden Bewegungen bzw. Relativbewegungen im zu behandelnden Bereich erfasst und nachregistriert.

2. Verfahren nach Anspruch 1, bei dem der Patientendatensatz mittels eines oder mehreren der folgenden Verfahren erstellt wird: Computertomographie (CT), Kernspintomographie (Magnetresonanztomographie, MRI), PET, SPECT, Ultraschallerfassung.

3. Verfahren nach Anspruch 1 oder 2, bei dem an zueinander beweglichen Körperstrukturen jeweils Markeranordnungen angebracht werden, so dass die Relativbewegungen dieser Strukturen zueinander erfasst werden.

4. Verfahren nach einem der Ansprüche 1 bis 3, bei dem der Bildinhalt der Röntgenaufnahme(n) computerunterstützt segmentiert und dabei einzelne, insbesondere zueinander bewegliche, Strukturen als getrennte Objekte erfasst und eingeordnet bzw. entsprechenden Objekten aus dem Patientendatensatz zugeordnet werden.

5. Verfahren nach Anspruch 4, bei dem die Segmentierung und Ein- bzw. Zuordnung automatisch erfolgt.

6. Verfahren nach einem der Ansprüche I bis 5, bei dem die aktuelle Registrierung des Patientendatensatzes durch Vergleich und Abgleich der Röntgenaufnahme(n) mit digital rekonstruierten Röntgenbildern (DRR's) aus dem Patientendatensatz erfolgt.

7. Verfahren nach einem der Ansprüche 1 bis 5, bei dem die aktuelle Registrierung des Patientendatensatzes durch Vergleich und Abgleich von Konturen aus der/den Röntgenaufnahme(n) mit Oberflächeninformationen aus dem Patientendatensatz erfolgt.

8. Verfahren nach einem der Ansprüche 1 bis 5, bei dem die aktuelle Registrierung des Patientendatensatzes mittels eines im Navigationssystem registrier- und verfolgbaren C-Bogen-Röntgengerät, das um eine horizontale Achse drehbar ist und einen isozentrischen Strahlengang aufweist, erfolgt.

9. Verfahren nach einem der Ansprüche 1 bis 8, **dadurch gekennzeichnet, dass** die Zuordnung von Strukturen aus dem Patientendatensatz und der/den Röntgenaufnahme(n) mittels einer dreidimensionalen Bildfusionstechnik über Grauwertabgleich erfolgt.

## Claims

1. A method for registering a patient data set obtained from an imaging process in camera navigation-supported surgery, comprising the following steps:
- producing a patient data set for the patient body or a part of it, by of a tomographic scan or an imaging process;
- applying at least one arrangement of markers, which may be identified and tracked in the navigation system, to at least one rigid body structure, once the patient has been brought into an operating room and during treatment;
- producing one or more x-ray images of the area to be treated by means of an x-ray device, the spatial position of said images in the navigation system being determined;
- currently registering the patient data set using the positional data obtained from the x-ray image, by means of computer-assisted assignment; and
- detecting and re-registering movements and/or relative movements in the area to be treated by tracking the arrangement(s) of markers in the navigation system.

2. The method as set forth in claim 1, wherein the patient data set is produced by means of one or more of the following methods: computer tomography (CT); nuclear spin tomography (magnetic resonance tomography, MRI); PET; SPECT; ultrasound detection.

3. The method as set forth in claim 1 or 2, wherein arrangements of markers are applied to each of the body structures moving relative to each other, such that the movements of said structures relative to each other can be detected.

4. The method as set forth in any one of claims 1 to 3, wherein the image content of the x-ray image(s) is segmented with the aid of a computer, thus detecting and arranging individual structures, in particular structures moving relative to each other, as separate objects or assigning them to corresponding objects from the patient data set.

5. The method as set forth in claim 4, wherein segmenting and arranging and/or assigning takes place automatically.

6. The method as set forth in any one of claims 1 to 5, wherein the patient data set is currently registered by comparing and adjusting the x-ray image(s) with digitally reconstructed radiograms (DRRs) from the patient data set.

7. The method as set forth in any one of claims 1 to 5, wherein the patient data set is currently registered by comparing and adjusting contours from the x-ray image(s) with surface information from the patient data set.

8. The method as set forth in any one of claims 1 to 5, wherein the patient data set is currently registered by means of a C-arc x-ray device which can be registered and tracked in the navigation system and rotated about a horizontal axis and which comprises an isocentric beam path.

9. The method as set forth in any one of claims 1 to 8, **characterised in that** the structures from the patient data set and the x-ray image(s) are assigned by means of a three-dimensional image fusion technique using grey-scale value adjustment.

## Revendications

1. Procédé d'enregistrement d'une série de données relatives à un patient en partant d'un procédé de visualisation lors d'interventions chirurgicales assistées par un système de navigation à caméra, comportant les étapes suivantes :
- on constitue une série de données pour un patient au moyen d'un balayage tomographique, respectivement d'un procédé de visualisation du corps du patient ou d'une partie de celui-ci,
- après amenée du patient en salle d'opération et lors du traitement, on applique sur au moins une structure fixe du corps au moins un dispositif de marqueur pouvant être identifé et suivi ou observé dans le système de navigation,
- au moyen d'un appareil à rayons X on prend une ou plusieurs radiographies de la zone à traiter, dont on détermine la position spatiale dans le système de navigation,
- au moyen d'un dispositif assisté par ordinateur, on enregistre actuellement la série des données du patient avec les données de position obtenues à partir des radiographies, et
- en observant ou suivant le ou les systèmes de marqueur dans le système de navigation, on saisit les mouvements, respectivement les mouvements relatifs dans la zone à traiter et on les enregistre ensuite.

2. Procédé selon la revendication, dans lequel la série des données du patient est établie au moyen d'un ou plusieurs des procédés suivants : tomographie assistée par ordinateur (CT), tomographie par spin nucléaire (tomographie par résonance magnétique, MRI), PET, SPECT, prise par ultra-sons.

3. Procédé selon la revendication 1 ou 2, dans lequel on applique sur des structures corporelles mobiles les unes par rapport aux autres chaque fois des dispositifs marqueurs de manière à saisir les mouvements relatifs de ces structures.

4. Procédé selon l'une des revendications 1 à 3, dans lequel le contenu d'image de la ou des radiographies est segmenté sous assistance d'ordinateur et ainsi des structures individuelles, en particulier mobiles les unes par rapport aux autres, sont saisies en tant qu'objets distincts et sont coordonnées ou classées, respectivement sont attribuées à des objets correspondants de la série des données du patient.

5. Procédé selon la revendication 4, dans lequel la segmentation et la coordination, respectivement l'attribution ont lieu automatiquement.

6. Procédé selon l'une des revendications 1 à 5, dans lequel l'enregistrement actuel de la série des données du patient a lieu par comparaison et ajustement de la ou des radiographies par rapport à des images radiographiques reconstruites par voie digitale (DRR's) à partir de la série des données du patient.

7. Procédé selon l'une des revendications 1 à 5, dans lequel l'enregistrement actuel de la série des données du patient a lieu par comparaison et ajustement des contours ou des profils en provenance de la ou des radiographies par rapport à des informations superficielles en provenance de la série des données du patient.

8. Procédé selon l'une des revendications 1 à 5, dans lequel l'enregistrement actuel de la série des données du patient a lieu au moyen d'un appareil à rayons X à courbure en C, se prêtant à l'enregistrement et à la poursuite, dans le système de navigation, capable de tourner autour d'un axe horizontal et présentant un parcours de rayonnement isocentrique.

9. Procédé selon l'une des revendications 1 à 8, **caractérisé en ce que** l'attribution des structures provenant de la série des données du patient et de la ou des radiographies a lieu au moyen d'une technique de diffusion d'images en trois dimensions en passant par un ajustement des valeurs de gris.
